(19) Europäisches Patentamt / European Patent Office / Office européen des brevets

(11) **EP 4 707 309 A1**

(12) **EUROPEAN PATENT APPLICATION**
published in accordance with Art. 153(4) EPC

(43) Date of publication:
**11.03.2026 Bulletin 2026/11**

(21) Application number: **24803454.8**

(22) Date of filing: **02.05.2024**

(51) International Patent Classification (IPC):
***C08F 22/40*** *(2006.01)* ***C07D 207/452*** *(2006.01)*
***H01M 10/052*** *(2010.01)* ***H01M 10/0565*** *(2010.01)*

(52) Cooperative Patent Classification (CPC):
Y02E 60/10

(86) International application number:
**PCT/JP2024/016899**

(87) International publication number:
**WO 2024/232342 (14.11.2024 Gazette 2024/46)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **08.05.2023 JP 2023076654**

(71) Applicant: **Sumitomo Chemical Company, Limited**
**Tokyo 103-6020 (JP)**

(72) Inventors:
- **NAKAJIMA, Hideto**
  **Niihama-shi, Ehime 792-8521 (JP)**
- **SUWA, Koki**
  **Niihama-shi, Ehime 792-8521 (JP)**

(74) Representative: **Vossius & Partner Patentanwälte Rechtsanwälte mbB**
**Siebertstraße 3**
**81675 München (DE)**

(54) **METHOD FOR PRODUCING ORGANIC LITHIUM SALT, COMPOSITION CONTAINING SAID ORGANIC LITHIUM SALT, AND POLYMER**

(57) The present disclosure provides a method for producing a compound (A1) represented by the following formula (A1), the method including a step of reacting a compound (D) represented by the following formula (D) with a compound (E1) represented by the following formula (E1) in the presence of at least two types of lithium salts.

[Chem. 1]

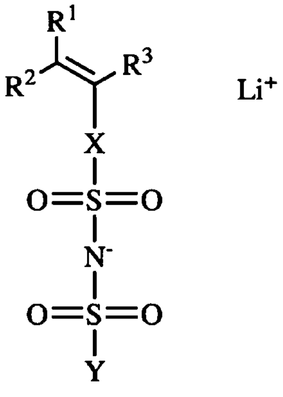

(A1)

(In formula (A1), $R^1$ is a hydrogen atom or a monovalent substituent, $R^3$ is a hydrogen atom or a monovalent substituent, Y is a halogen atom or a monovalent organic group having 1 to 20 carbon atoms, and X and $R^2$ satisfy the following (1) or (2):
(1) X is a divalent organic group having 1 to 20 carbon atoms, and $R^2$ is a monovalent organic group.
(2) $R^2$ and X are taken together to form a trivalent group.)

[Chem. 2]

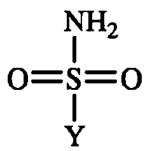

(D)

[Chem. 3]

EP 4 707 309 A1

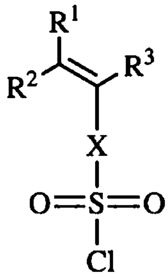

(E1)

## Description

### Technical Field

The present disclosure relates to a method for producing an organic lithium salt, a composition containing said organic lithium salt, and a polymer.

### Background Art

Organic lithium salts are used in many fields (Patent Literature 1). For example, an organic lithium salt having an ethylenically unsaturated group is used as a raw material (monomer) for producing a polymer used as a single-ion conductor for conducting lithium ions in a lithium ion battery.

### Citation List

### Patent Literature

Patent Literature 1: International Publication No. WO 2018/176134

### Summary of Invention

### Technical Problem

Here, an organic lithium salt may contain cations other than lithium ions as impurities. For example, in applications where lithium ions are directly linked to the function, such as in a lithium ion battery that performs charging and discharging by the movement of lithium ions between a positive electrode and a negative electrode, it is preferable to increase the concentration of lithium ions among all cations contained in an electrolyte or the like, as this leads to an improvement in performance.

The present disclosure has been made in view of the above circumstances, and an object thereof is to provide a method for producing an organic lithium salt with a low content of cations other than lithium ions. Another object of the present disclosure is to provide a composition that contains an organic lithium salt and has an excellent lithium ion transference number.

### Solution to Problem

[1] A method for producing a compound (A1) represented by the following formula (A1), the method including:

a step of reacting a compound (D) represented by the following formula (D) with a compound (E1) represented by the following formula (E1) in the presence of at least two types of lithium salts.

[Chem. 1]

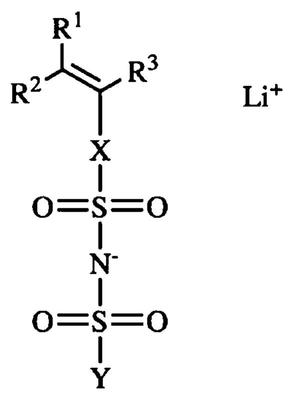

(A1)

(In formula (A1), $R^1$ is a hydrogen atom or a monovalent substituent, $R^3$ is a hydrogen atom or a monovalent

substituent, Y is a halogen atom or a monovalent organic group having 1 to 20 carbon atoms, and X and $R^2$ satisfy the following (1) or (2):

(1) X is a divalent organic group having 1 to 20 carbon atoms, and $R^2$ is a monovalent organic group.
(2) $R^2$ and X are taken together to form a trivalent group.)

[Chem. 2]

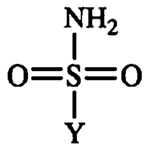

(D)

[Chem. 3]

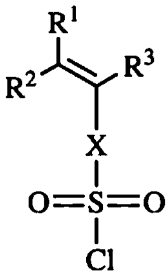

(E1)

[2] The method for producing according to [1], wherein the compound (A1) includes a compound (A2) represented by the following formula (A2).

[Chem. 4]

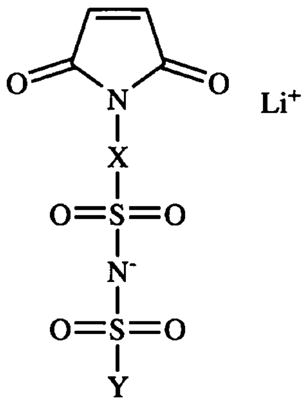

(A2)

[3] A method for producing a compound (A2) represented by the following formula (A2), the method including: a step of reacting a compound (C) represented by the following formula (C) in a solvent in the presence of a lithium-containing base.

[Chem. 5]

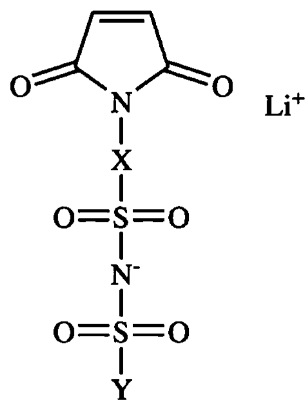

(A2)

(In formula (A2), X is a divalent organic group having 1 to 20 carbon atoms, and Y is a halogen atom or a monovalent organic group having 1 to 20 carbon atoms.)

[Chem. 6]

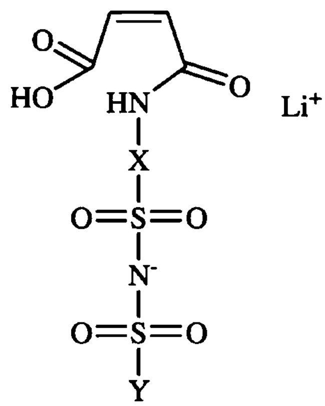

(C)

[4] The method for producing according to [3], wherein the lithium-containing base includes lithium acetate.

[5] The method for producing according to [3] or [4], wherein the solvent includes acetic anhydride.

[6] A composition including a compound (A) represented by the following formula (A), wherein a proportion of the compound (A) is 50 mol% or more with respect to a total amount of substance of the compound (A) and a compound (B) represented by the following formula (B) in the composition.

[Chem. 7]

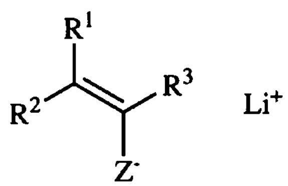

(A)

(In formula (A), $R^1$ is a hydrogen atom or a monovalent substituent, $R^3$ is a hydrogen atom or a monovalent substituent, and $R^2$ and $Z^-$ satisfy the following (3) or (4):

(3) $R^2$ is a hydrogen atom or a monovalent substituent, and $Z^-$ is a monovalent group having a monovalent anionic functional group.

(4) $R^2$ and $Z^-$ are taken together to form a divalent group having a monovalent anionic functional group.)

[Chem. 8]

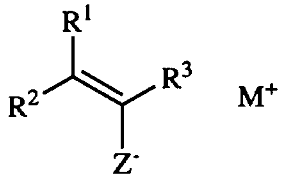

(B)

(In formula (B), $R^1$ to $R^3$ and $Z^-$ are the same as $R^1$ to $R^3$ and $Z^-$ in formula (A), respectively, and $M^+$ is a monovalent cation other than $H^+$ and $Li^+$.)

[7] The composition according to [6], wherein the compound (A) includes a compound (A1) represented by the following formula (A1), and the compound (B) includes a compound (B1) represented by the following formula (B1).

[Chem. 9]

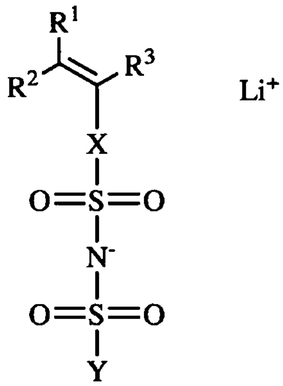

(A1)

(In formula (A1), Y is a halogen atom or a monovalent organic group having 1 to 20 carbon atoms, and X and $R^2$ satisfy the following (1) or (2):

(1) X is a divalent organic group having 1 to 20 carbon atoms, and $R^2$ is a monovalent organic group.
(2) $R^2$ and X are taken together to form a trivalent group.)

[Chem. 10]

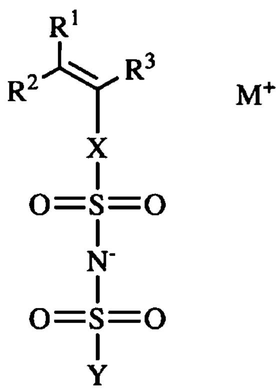

(B1)

(In formula (B1), X and Y are the same as X and Y in formula (A1), respectively.)

[8] The composition according to [6] or [7], wherein the compound (A) includes a compound (A2) represented by the following formula (A2), and the compound (B) includes a compound (B2) represented by the following formula (B2).

[Chem. 11]

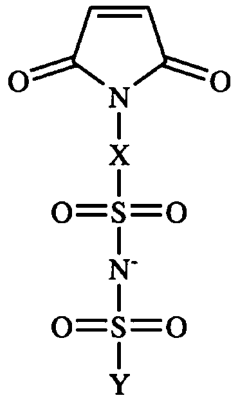 $Li^+$ (A2)

(In formula (A2), X is a divalent organic group having 1 to 20 carbon atoms, and Y is a halogen atom or a monovalent organic group having 1 to 20 carbon atoms.)

[Chem. 12]

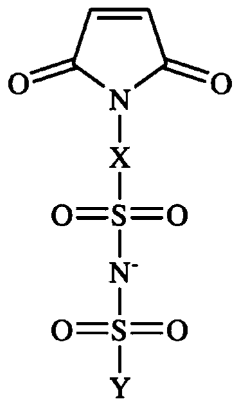 $M^+$ (B2)

(In formula (B2), X and Y are the same as X and Y in formula (A2), respectively.)

[9] The composition according to any one of [6] to [8], which is a monomer composition for forming a polymer for an electrolyte of a lithium ion battery.

[10] A polymer including a structural unit (PA) represented by the following formula (PA), wherein a proportion of the structural unit (PA) is 50 mol% or more with respect to a total amount of substance of the structural unit (PA) and a structural unit (PB) represented by the following formula (PB) in the polymer.

[Chem. 13]

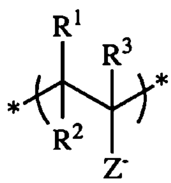 $Li^+$ (PA)

(In formula (PA), $R^1$ is a hydrogen atom or a monovalent substituent, $R^3$ is a hydrogen atom or a monovalent substituent, and $R^2$ and $Z^-$ satisfy the following (3) or (4):

(3) $R^2$ is a hydrogen atom or a monovalent substituent, and $Z^-$ is a monovalent group having a monovalent anionic functional group.

(4) $R^2$ and $Z^-$ are taken together to form a divalent group having a monovalent anionic functional group.)

[Chem. 14]

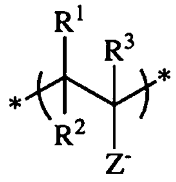

M+ (PB)

(In formula (PB), $R^1$ to $R^3$ and $Z^-$ are the same as $R^1$ to $R^3$ and $Z^-$ in formula (PA), respectively, and $M^+$ is a monovalent cation other than $H^+$ and $Li^+$.)

[11] The polymer according to [10], wherein the structural unit (PA) includes a structural unit (PA1) represented by the following formula (PA1), and the structural unit (PB) includes a structural unit (PB1) represented by the following formula (PB1).

[Chem. 15]

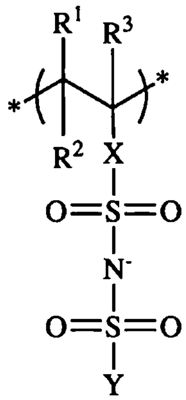

Li+ (PA1)

(In formula (PA1), Y is a halogen atom or a monovalent organic group having 1 to 20 carbon atoms, and X and $R^2$ satisfy the following (1) or (2):

(1) X is a divalent organic group having 1 to 20 carbon atoms, and $R^2$ is a monovalent organic group.
(2) $R^2$ and X are taken together to form a trivalent group.)

[Chem. 16]

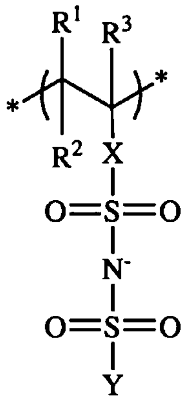

M+ (PB1)

(In formula (PB1), X and Y are the same as X and Y in formula (PA1), respectively.)

[12] The polymer according to [10] or [11], wherein the structural unit (PA) includes a structural unit (PA2) represented

by the following formula (PA2), and the structural unit (PB) includes a structural unit (PB2) represented by the following formula (PB2).

[Chem. 17]

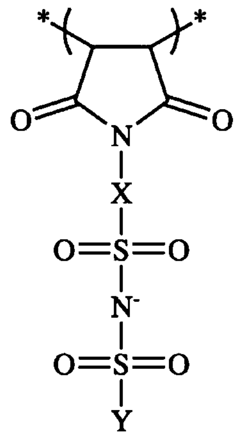

$Li^+$ (PA2)

(In formula (PA2), X is a divalent organic group having 1 to 20 carbon atoms, and Y is a halogen atom or a monovalent organic group having 1 to 20 carbon atoms.)

[Chem. 18]

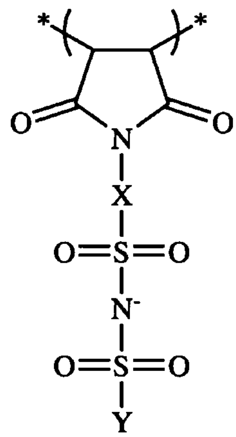

$M^+$ (PB2)

(In formula (PB2), X and Y are the same as X and Y in formula (PA2), respectively.)
[13] An electrolyte composition including the polymer according to any one of [10] to [12].
[14] A battery including the electrolyte composition according to [13].

## Advantageous Effects of Invention

According to the present disclosure, it is possible to provide a method for producing an organic lithium salt with a low content of cations other than lithium ions. According to the present disclosure, it is also possible to provide a composition that contains an organic lithium salt and has an excellent lithium ion transference number.

## Brief Description of Drawings

Fig. 1 is a diagram showing the measurement results of the lithium ion transference number for an electrolyte composition containing the copolymer of Example A1.
Fig. 2 is a diagram showing the measurement results of the lithium ion transference number for an electrolyte composition containing the copolymer of Comparative Example A1.

## Description of Embodiments

(Composition containing organic lithium salt)

The composition of the present embodiment includes a compound (A) represented by the following formula (A), and a proportion of the compound (A) is 50 mol% or more with respect to a total amount of substance of the compound (A) and a compound (B) represented by the following formula (B) in the composition. The organic lithium salt contained in the composition of the present embodiment is a compound having an ethylenically unsaturated group and an anionic functional group with a lithium ion as a counter cation. The proportion of the compound (A) may be 55 mol% or more, may be 60 mol% or more, may be 65 mol% or more, may be 70 mol% or more, and may be 75 mol% or more.

[Chem. 19]

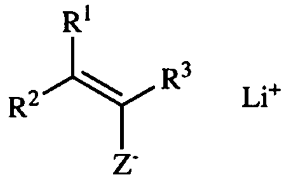

(A)

(In formula (A), $R^1$ is a hydrogen atom or a monovalent substituent, $R^3$ is a hydrogen atom or a monovalent substituent, and $R^2$ and $Z^-$ satisfy the following (3) or (4):

(3) $R^2$ is a hydrogen atom or a monovalent substituent, and $Z^-$ is a monovalent group having a monovalent anionic functional group.

(4) $R^2$ and $Z^-$ are taken together to form a divalent group having a monovalent anionic functional group.)

[Chem. 20]

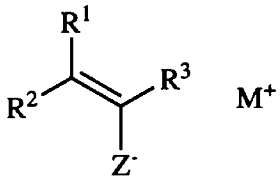

(B)

(In formula (B), $R^1$ to $R^3$ and $Z^-$ are the same as $R^1$ to $R^3$ and $Z^-$ in formula (A), respectively, and $M^+$ is a monovalent cation other than $H^+$ and $Li^+$.)

That is, in the composition of the present embodiment, the content of the compound (A) is 50 mol% or more with respect to the total amount of substance of the compound (A) and the compound (B), which differs from the compound (A) only in the type of cation. In the composition of the present embodiment, the content of the compound (B) may be 0 (the composition of the present embodiment may not contain the compound (B)). In the composition in the present embodiment, the compound (A) may be one type of compound represented by formula (A), and the compound (B) may be a compound corresponding to the above compound (A) (a compound in which $Li^+$ of the compound (A) is replaced with $M^+$). The salt contained in the composition of the present embodiment may consist of the compound (A) and the compound (B), or may consist only of the compound (A). When the composition of the present embodiment consists only of the compound (A), the composition can be read as a compound. The composition may contain components other than the compound (A) and the compound (B) (excluding salts) as long as they do not depart from the spirit of the present disclosure. The present disclosure can provide a lithium salt with a high lithium concentration (a lithium salt composition with a low proportion of compounds in which $Li^+$ is replaced with $M^+$) that could not be obtained conventionally.

Among $R^1$ to $R^3$, one or more may be a hydrogen atom, and one or two may be hydrogen atoms.

When one or more of $R^1$ to $R^3$ is a monovalent substituent, examples of the monovalent substituent include a monovalent organic group, a monovalent electron-withdrawing group (excluding the above monovalent organic group), and the like. Examples of the monovalent organic group include an organic group having 1 to 20 carbon atoms. The number of carbon atoms contained in the monovalent organic group may be 1 to 10, 1 to 8, or 1 to 5. The monovalent organic group is not particularly limited, and may be a hydrocarbon group or a group having a heteroatom, and may have a heterocyclic ring. More specifically, examples include a hydrocarbon group, a group having a chemical structure in which

one or more carbon atoms (methylene groups) in the hydrocarbon group are substituted by a linking group of -O-, -S-, -C(=O)-, -C(=O)O-, or -C(=O)NR-, and the like. R in the -C(=O)NR- may be a hydrogen atom or a hydrocarbon group having 1 to 5 carbon atoms. When there are a plurality of linking groups, the linking groups are not adjacent to each other. The monovalent organic group may have a substituent that substitutes a hydrogen atom bonded to a carbon atom. The substituent may be a monovalent substituent, and examples thereof include a halogen atom and the like. The halogen atom may be any of F, Cl, Br, and I, and may be F. The hydrocarbon group is not particularly limited, and may be an aliphatic hydrocarbon group or an aromatic hydrocarbon group. The aliphatic hydrocarbon group may be any of a linear hydrocarbon group, a branched hydrocarbon group, and a cyclic hydrocarbon group. The hydrocarbon group may be either a saturated hydrocarbon group or an unsaturated hydrocarbon group.

In the present specification, an aromatic hydrocarbon group is a group including an aromatic moiety, and may have an aliphatic moiety. In the present specification, a cyclic hydrocarbon group is a group including a cyclic hydrocarbon moiety, and may include a linear or branched hydrocarbon moiety.

Examples of the monovalent electron-withdrawing group include a halogen atom, a sulfonic acid group or a salt thereof, a sulfonic acid ester, a nitro group, a nitrile group, a carbonyl group, an ester group, an amide group, and the like. The halogen atom may be any of F, Cl, Br, and I, and may be F.

$Z^-$ is a monovalent group having a monovalent anionic functional group. Examples of the monovalent anionic functional group include a carboxylate group ($-COO^-$), a conjugated anion of a sulfonic acid group ($-SO_3^-$), a conjugated anion of a sulfonylimide group ($-SO_2-N^--SO_2-$), a conjugated anion of a phenolic hydroxyl group ($-C_6H_5-O^-$), and the like. The conjugated anion of a sulfonylimide group may be included in an anionic functional group represented by $-SO_2-N^--SO_2-Y$ using Y described later. $Z^-$ may be, for example, a monovalent organic group having 1 to 30 carbon atoms, or may be the anionic functional group itself. The number of carbon atoms that the monovalent organic group has may be 1 to 25, may be 2 to 20, and may be 3 to 15.

As the compound of formula (A), a compound having a carboxylate group may be an unsaturated lithium carboxylate, may be an unsaturated lithium monocarboxylate, and may be lithium (meth)acrylate. As the compound of formula (A), a compound having a conjugated anion of a phenolic hydroxyl group may be a compound having a vinyl group and a benzene skeleton, a naphthalene skeleton, or an anthracene skeleton, and may be lithium 4-vinylphenolate, lithium 3-vinylphenolate, or lithium 2-vinylphenolate, and may be lithium 4-vinylphenolate.

When $R^2$ and $Z^-$ are taken together to form a divalent group having a monovalent anionic functional group, examples of the compound (A) include a compound (A') represented by the following formula (A').

[Chem. 21]

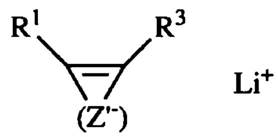

(A')

(In the formula, $Z'^-$ is a divalent group having a monovalent anionic functional group.)

Examples of the monovalent anionic functional group that $Z'^-$ has include the monovalent anionic functional groups that the above-mentioned $Z^-$ has. $Z'^-$ may be, for example, a divalent organic group having 1 to 30 carbon atoms. The number of carbon atoms that the divalent organic group has may be 1 to 25, may be 2 to 20, and may be 3 to 15. $Z'^-$ forms a ring together with the two carbon atoms forming an unsaturated bond in formula (A'). The number of ring members of the ring may be 4 to 10, may be 4 to 8, and may be 5 or 6. $Z'^-$ may have a cyclic imide structure.

Regarding the compound (B), $M^+$ is a monovalent cation other than $H^+$ and $Li^+$. Examples of the monovalent cation include a metal cation, an organic cation, an inorganic cation other than a metal cation such as $NH_4^+$, and the like. Examples of the metal cation include an alkali metal ion and the like. The alkali metal ion may be any of $Na^+$, $K^+$, $Rb^+$, and $Cs^+$. The monovalent metal cation may be a complex cation. Examples of the monovalent organic cation include cations containing N, P, S, I, etc. having a positive formal charge, and specific examples include a quaternary organic ammonium ion, a quaternary phosphonium ion, a sulfonium ion, an iodonium ion, and the like. $M^+$ may include $Na^+$.

When the compound (A) is the compound (A'), the corresponding compound (B) is a compound (B') represented by the following formula (B').

[Chem. 22]

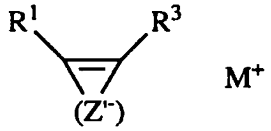 (B')

(In formula (B'), Z'⁻ is the same as Z'⁻ in formula (A').)

The composition may include one or two or more types of the compound (A). The content of the compound (A) in the composition may be 40 mass% or more, may be 50 mass% or more, may be 55 mass% or more, may be 60 mass% or more, may be 65 mass% or more, and may be 70 mass% or more, with respect to the total amount of the composition.

The compound (A) may include a compound (A1) represented by the following formula (A1). In this case, the compound (B) corresponding to the compound (A1) is a compound (B1) represented by the following formula (B1).

[Chem. 23]

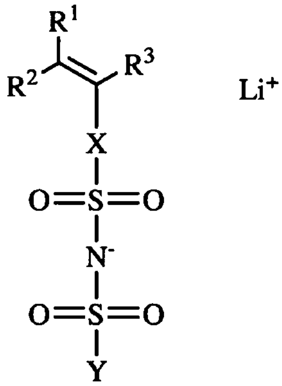 (A1)

(In formula (A1), Y is a halogen atom or a monovalent organic group having 1 to 20 carbon atoms, and X and $R^2$ satisfy the following (1) or (2):

(1) X is a divalent organic group having 1 to 20 carbon atoms, and $R^2$ is a monovalent organic group.
(2) $R^2$ and X are taken together to form a trivalent group.)

[Chem. 24]

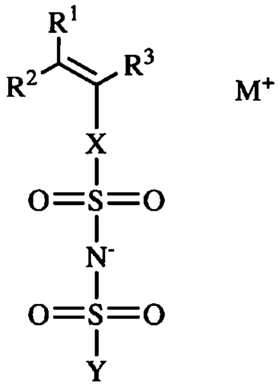 (B1)

(In formula (B1), X and Y are the same as X and Y in formula (A1), respectively.)

X is not particularly limited, and may be a hydrocarbon group or a group having a heteroatom, and may have a heterocyclic ring. More specifically, examples of X include divalent groups such as a hydrocarbon group, a group having a chemical structure in which one or more carbon atoms (methylene groups) in the hydrocarbon group are substituted by a linking group of -O-, -S-, -C(=O), -C(=O)O-, or -C(=O)NR-, and the like. R in the -C(=O)NR- may be a hydrogen atom or a hydrocarbon group having 1 to 5 carbon atoms. When there are a plurality of linking groups, the linking groups are not

adjacent to each other. The divalent group may have a substituent that substitutes a hydrogen atom bonded to a carbon atom. The substituent may be a monovalent substituent, and examples thereof include a halogen atom and the like. The hydrocarbon group is not particularly limited, and may be an aliphatic hydrocarbon group or an aromatic hydrocarbon group. The aliphatic hydrocarbon group may be any of a linear hydrocarbon group, a branched hydrocarbon group, and a cyclic hydrocarbon group. The hydrocarbon group may be either a saturated hydrocarbon group or an unsaturated hydrocarbon group.

The number of carbon atoms that X has may be 1 to 15, may be 2 to 10, and may be 3 to 8. X may be a group having an aromatic ring, and may be a group having an aromatic carbocycle such as a benzene ring. A substituent may be bonded to a carbon atom that is a ring member of the carbocycle, and the substituent may be a monovalent substituent. The monovalent substituent may be a substituent such as a substituted or unsubstituted alkyl group or an electron-withdrawing group. The hydrocarbon group as X is preferably a phenylene group, an alkylene group having 1 to 8 carbon atoms, a polyoxyalkylene group, or a group in which some or all of the hydrogen atoms bonded to the carbon atoms thereof are substituted by a substituent such as a halogen atom such as a fluorine atom, and may be a phenylene group or a substituted phenylene group substituted with an alkyl group, a halogen atom, an electron-withdrawing group, or the like. Examples of the electron-withdrawing group include a halogen atom, a sulfonic acid group or a salt thereof, a sulfonic acid ester, a nitro group, a nitrile group, a carbonyl group, an ester group, an amide group, and the like.

When Y is a monovalent organic group, the organic group is not particularly limited, and may be a hydrocarbon group or a group having a heteroatom, and may have a heterocyclic ring. More specifically, examples of Y include monovalent groups such as a hydrocarbon group, a group having a chemical structure in which one or more carbon atoms (methylene groups) in the hydrocarbon group are substituted by a linking group of -O-, -S-, -C(=O), -C(=O)O-, or -C(=O)NR-, and the like. R in the -C(=O)NR- may be a hydrogen atom or a hydrocarbon group having 1 to 5 carbon atoms. When there are a plurality of linking groups, the linking groups are not adjacent to each other. The monovalent group may have a substituent that substitutes a hydrogen atom bonded to a carbon atom. The substituent may be a monovalent substituent, and examples thereof include a halogen atom and the like. The hydrocarbon group is not particularly limited, and may be an aliphatic hydrocarbon group or an aromatic hydrocarbon group. The aliphatic hydrocarbon group may be any of a linear hydrocarbon group, a branched hydrocarbon group, and a cyclic hydrocarbon group. The hydrocarbon group may be either a saturated hydrocarbon group or an unsaturated hydrocarbon group.

The number of carbon atoms that Y has may be 1 to 25, may be 1 to 20, may be 1 to 15, may be 1 to 10, may be 1 to 8, may be 1 to 5, and may be 1 to 3. The hydrocarbon group as Y may be a substituted or unsubstituted phenyl group, an alkyl group having 1 to 5 carbon atoms, or one in which some or all of the hydrogen atoms bonded to the carbon atoms of these groups are substituted with a halogen atom such as a fluorine atom, may be a fluorinated alkyl group having 1 to 5 carbon atoms, and may be a fluorinated alkyl group having 1 to 3 carbon atoms such as a trifluoromethyl group. The fluorinated alkyl group may be a fully fluorinated alkyl group. When Y is a halogen atom, the halogen atom may be a fluorine atom or a chlorine atom, and may be a fluorine atom.

When $R^2$ and X are taken together to form a trivalent group, the compound represented by formula (A1) may be a compound (A1') represented by the following formula (A1'). In this case, the corresponding compound (B1) is a compound (B1') represented by the following formula (B1').

[Chem. 25]

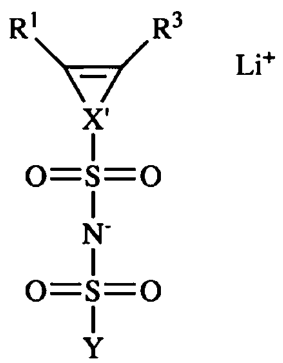

(A1')

(In formula (A1'), X' is a trivalent organic group.)

[Chem. 26]

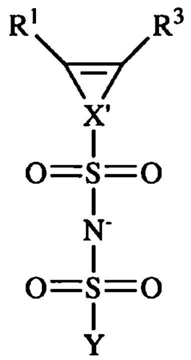

M+ (B1')

(In formula (B1'), X' is the same as X' in formula (A1').)

X' has a ring including the two carbon atoms forming an unsaturated bond in formula (A1'). The number of ring members of the ring may be 4 to 10, may be 4 to 8, and may be 5 or 6. The ring may have a cyclic imide structure. The structure other than the ring contained in X' is not particularly limited, and may be a divalent group or a covalent bond bonded to X' and the S atom of the sulfonylimide group, and the divalent group may be a hydrocarbon group or a group having a heteroatom, and may have a heterocyclic ring. More specifically, examples of X include divalent groups such as a hydrocarbon group, a group having a chemical structure in which one or more carbon atoms (methylene groups) in the hydrocarbon group are substituted by a linking group of -O-, -S-, -C(=O)-, or -C(=O)O-, and the like. When there are a plurality of linking groups, the linking groups are not adjacent to each other. The trivalent group may have a substituent that substitutes a hydrogen atom bonded to a carbon atom. The substituent may be a monovalent substituent, and examples thereof include a halogen atom and the like. The hydrocarbon group is not particularly limited, and may be an aliphatic hydrocarbon group or an aromatic hydrocarbon group. The aliphatic hydrocarbon group may be any of a linear hydrocarbon group, a branched hydrocarbon group, and a cyclic hydrocarbon group. The hydrocarbon group may be either a saturated hydrocarbon group or an unsaturated hydrocarbon group.

The compound (A) may include a compound (A2) represented by the following formula (A2). In this case, the compound (B) corresponding to the compound (A2) is a compound (B2) represented by the following formula (B2).

[Chem. 27]

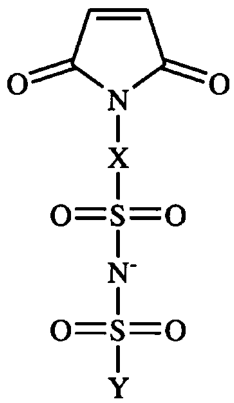

Li+ (A2)

(In formula (A2), X is a divalent organic group having 1 to 20 carbon atoms, and Y is a halogen atom or a monovalent organic group having 1 to 20 carbon atoms.)

[Chem. 28]

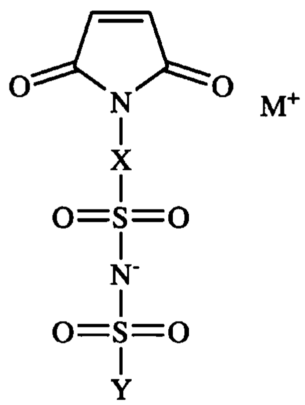

(B2)

(In formula (B2), X and Y are the same as X and Y in formula (A2), respectively.)

Examples of X in formula (A2) include those exemplified as X in formula (A1). Examples of Y in formula (A2) include those exemplified as Y in formula (A1).

In formula (A2), X may be bonded to one or both of the nitrogen atom of the maleimide group and the sulfur atom of the sulfonyl group via a carbon atom that X has.

The composition of the present embodiment can be used, for example, to produce a polymer (lithium-containing polymer) by a polymerization reaction of the ethylenically unsaturated group of the compound (A). That is, the composition of the present embodiment can be used as a monomer composition, and may be a monomer composition for forming a polymer for an electrolyte of a lithium ion battery. The polymerization reaction may be a radical addition polymerization reaction, a radical ring-opening polymerization reaction, a cationic polymerization reaction, or an anionic polymerization reaction, and may be a radical addition polymerization.

The compound (A) may be polymerized alone, or may be subjected to a polymerization reaction together with other monomers to form a copolymer. Examples of other monomers include compounds having an ethylenically unsaturated group other than the compound (A) and the compound (B). Examples of other monomers include unsaturated fatty acids such as (meth)acrylic acid and maleic acid, unsaturated fatty acid esters such as (meth)acrylic acid esters, vinyl ether compounds such as isobutyl vinyl ether and n-dodecyl vinyl ether, vinyl ester compounds such as vinyl acetate, olefin compounds such as ethylene and propylene, aromatic vinyl compounds such as styrene or styrene derivatives, unsaturated aldehydes, acrylonitrile or its derivatives, acrylamide or its derivatives, and the like.

The polymer can be produced by subjecting a polymerizable composition to a polymerization reaction. The polymerizable composition includes the composition including the compound (A) of the present embodiment, and may optionally include other components such as other monomers and a radical polymerization initiator.

When m is the number of moles of the compound (A) with respect to the number of moles of all monomers contained in the polymerizable composition, m may be 0.1 to 0.9, may be 0.2 to 0.8, may be 0.3 to 0.7, and may be 0.4 to 0.6. The content of the structural unit (PA) with respect to the total amount of monomers contained in the polymerizable composition may be 10 to 90 mass%, and may be 25 to 70 mass%.

The radical polymerization initiator may be either a thermal initiator or a photoinitiator. For example, examples of the thermal initiator include azo-based initiators such as 2,2-azobis(isobutyronitrile) (AIBN); 2,2-azobis(2-methylbutyronitrile) (AMBN), 2,2-azobis(2,4-dimethylvaleronitrile) (ADVN), 1, 1-azobis(1-cyclohexanecarbonitrile) (ACHN, V-40), and dimethyl-2,2-azobisisobutyrate (MAIB); and organic peroxides such as dibenzoyl peroxide, di-8,5,5-trimethylhexanoyl peroxide, dilauroyl peroxide, didecanoyl peroxide, and di(2,4-dichlorobenzoyl) peroxide. Examples of the photoinitiator include oxime-based compounds, metallocene-based compounds, acylphosphine-based compounds, aminoacetophenone compounds, and the like. One or two or more types of initiators may be used.

The polymerizable composition may include a chain transfer agent such as carbon tetrachloride. The polymerizable composition may optionally include a crosslinking agent. The polymerizable composition may also include a solvent.

(Lithium-containing polymer)

The polymer of the present embodiment is a lithium-containing polymer that includes an anionic functional group and lithium ions as counter cations for the anionic functional group. The polymer includes a structural unit (PA) represented by the following formula (PA), and a proportion of the structural unit (PA) is 50 mol% or more with respect to a total amount of substance of the structural unit (PA) and a structural unit (PB) represented by the following formula (PB) in the polymer. The

proportion of the structural unit (PA) may be 55 mol% or more, may be 60 mol% or more, may be 65 mol% or more, may be 70 mol% or more, and may be 75 mol% or more.

[Chem. 29]

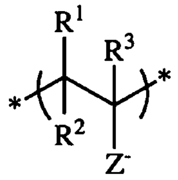

$Li^+$ (PA)

(In formula (PA), $R^1$ is a hydrogen atom or a monovalent substituent, $R^3$ is a hydrogen atom or a monovalent substituent, and $R^2$ and $Z^-$ satisfy the following (3) or (4):

(3) $R^2$ is a hydrogen atom or a monovalent substituent, and $Z^-$ is a monovalent group having a monovalent anionic functional group.
(4) $R^2$ and $Z^-$ are taken together to form a divalent group having a monovalent anionic functional group.)

[Chem. 30]

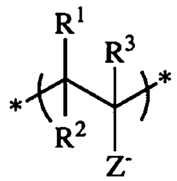

$M^+$ (PB)

(In formula (PB), $R^1$ to $R^3$ and $Z^-$ are the same as $R^1$ to $R^3$ and $Z^-$ in formula (PA), respectively, and M is a monovalent cation other than $Li^+$.)

Regarding the structural unit (PB), $M^+$ is a monovalent cation other than $H^+$ and $Li^+$. Examples of the monovalent cation include a metal cation, an organic cation, an inorganic cation other than a metal cation such as $NH_4^+$, and the like. Examples of the metal cation include an alkali metal ion and the like. The alkali metal ion may be any of $Na^+$, $K^+$, $Rb^+$, and $Cs^+$. The monovalent metal cation may be a complex cation. Examples of the monovalent organic cation include cations containing N, P, S, I, etc. having a positive formal charge, and specific examples include a quaternary organic ammonium ion, a quaternary phosphonium ion, a sulfonium ion, an iodonium ion, and the like. $M^+$ may include $Na^+$.

Such a polymer can be produced from the composition including the compound (A). The polymer may include structural units other than the structural unit (PA) and the structural unit (PB), and examples of such structural units include structural units derived from the other monomers mentioned above (structural units obtained by radical polymerization of the other monomers).

Examples of $R^1$ to $R^3$ and $Z^-$ in formula (PA) include those exemplified as $R^1$ to $R^3$ and $Z^-$ in formula (A), respectively.

When $R^2$ and $Z^-$ are taken together to form a divalent group having a monovalent anionic functional group, examples of the structural unit (PA) include a structural unit (PA') represented by the following formula (PA'). When the structural unit (PA) is the structural unit (PA'), the corresponding structural unit (PB) is a structural unit (PB') represented by the following formula (PB'). In the present specification, unless otherwise specified, * in a chemical formula representing a structural unit denotes the position at which the structural unit is bonded to another structural unit.

[Chem. 31]

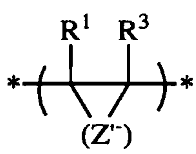

$Li^+$ (PA')

(In formula (PA'), $Z'^-$ is a divalent group having a monovalent anionic functional group.)

[Chem. 32]

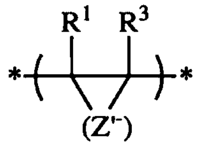

$M^+$ (PB')

(In formula (PB'), $Z'^-$ is the same as $Z'^-$ in formula (PA').)

Examples of $Z'^-$ in formula (PA') include those exemplified as $Z'^-$ in formula (A').

The structural unit (PA) may include a structural unit (PA1) represented by the following formula (PA1). In this case, the structural unit (PB) corresponding to the structural unit (PA1) is a structural unit (PB1) represented by the following formula (PB1).

[Chem. 33]

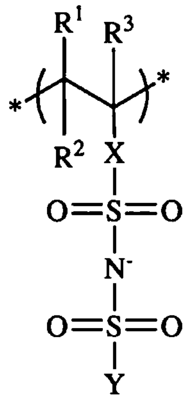

$Li^+$ (PA1)

(In formula (PA1), Y is a halogen atom or a monovalent organic group having 1 to 20 carbon atoms, and X and $R^2$ satisfy the following (1) or (2):

(1) X is a divalent organic group having 1 to 20 carbon atoms, and $R^2$ is a monovalent organic group.
(2) $R^2$ and X are taken together to form a trivalent group.)

[Chem. 34]

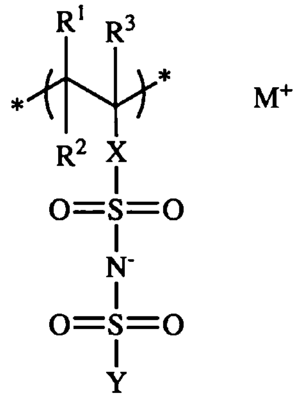

(PB1)

(In formula (PB1), X and Y are the same as X and Y in formula (PA1), respectively.)

Examples of X and Y in formula (PA1) include those exemplified as X and Y in formula (A1), respectively.

When $R^2$ and X are taken together to form a trivalent group, the structural unit represented by formula (PA1) may be a structural unit (A1') represented by the following formula (PA1'). In this case, the corresponding structural unit (PB1) is a structural unit (PB1') represented by the following formula (PB1').

[Chem. 35]

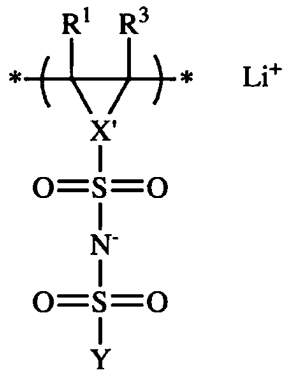

(PA1')

(In formula (PA1'), X' is a trivalent organic group.)

[Chem. 36]

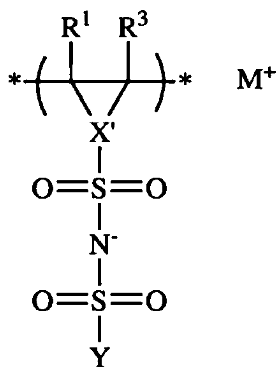

(PB1')

(In formula (PB1'), X' is the same as X' in formula (PA1').)

Examples of X' in formula (PA1') include those exemplified as X' in formula (A1').

The structural unit (PA) may include a structural unit (PA2) represented by the following formula (PA2). In this case, the structural unit (PB) corresponding to the structural unit (PA2) is a structural unit (PB2) represented by the following formula (PB2).

[Chem. 37]

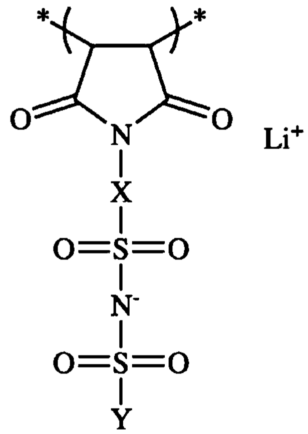

(PA2)

(In formula (PA2), X is a divalent organic group having 1 to 20 carbon atoms, and Y is a halogen atom or a monovalent organic group having 1 to 20 carbon atoms.)

[Chem. 38]

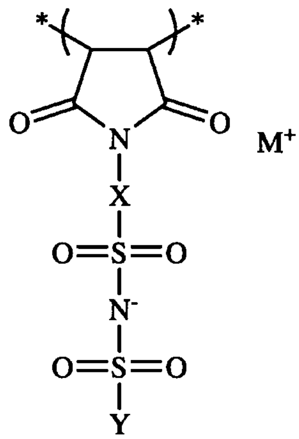

(PB2)

(In formula (PB2), X and Y are the same as X and Y in formula (PA2), respectively.)

Examples of X and Y in formula (PA2) include those exemplified as X and Y in formula (A2), respectively.

When m is the number of moles of the structural unit (PA) with respect to the number of moles of all structural units contained in the polymer, m may be 0.1 to 0.9, may be 0.2 to 0.8, may be 0.3 to 0.7, and may be 0.4 to 0.6. The content of the structural unit (PA) with respect to the total amount of the polymer may be 10 to 90 mass%, and may be 25 to 70 mass%.

The number average molecular weight (Mn) of the polymer may be 5,000 to 400,000, may be 8,000 to 200,000, may be 10,000 to 150,000, and may be 10,000 to 100,000. The weight average molecular weight (Mw) of the polymer may be 5,000 to 600,000, may be 10,000 to 450,000, may be 20,000 to 200,000, and may be 20,000 to 100,000. The molecular weight distribution (Mw/Mn) of the polymer may be 1.0 to 5.0, may be 1.2 to 3.0, and may be 1.3 to 2.5. The number average molecular weight and weight average molecular weight of the polymer can be measured, for example, by gel permeation chromatography.

The gel permeation chromatography (GPC) shall be measured under the conditions shown below. For the creation of a calibration curve, a standard sample of polymethyl methacrylate (manufactured by Polymer Laboratories, Mn of 800 to 2,200,000) shall be used as a standard substance.

<GPC Measurement Conditions>

Apparatus: High-performance liquid chromatograph manufactured by JASCO Corporation

PU-2080 precision pump
RI-2031 refractive-index detector
UV-2075 UV/vis detector

Column: Shodex KF-805L (exclusion limit: $4\times10^6$, particle size: 10 μm, pore size: 5000 Å, inner diameter: 0.8 cm, length: 30 cm)
Temperature: 40°C
Solvent: N, N-Dimethylformamide (DMF)
Flow rate: 1.0 mL/min
Back pressure: 3.0 MPa
Detection: RI
Sample concentration: 0.5 mass% DMF solution
Injection volume: 10 μL

The polymer of the present embodiment can be used, for example, as a lithium ion conductor. Since the polymer of the present embodiment has a large proportion of lithium ions relative to cations other than lithium ions, it can be used as a material for at least one of a positive electrode, a negative electrode, and an electrolyte of a lithium ion battery, and may be used for an electrolyte.

The lithium ion battery of the present embodiment includes a positive electrode, a negative electrode, and an electrolyte disposed between the positive electrode and the negative electrode. The positive electrode may be one in which a layer including a positive electrode material containing a positive electrode active material is formed on a current collector. The positive electrode material may include, in addition to the positive electrode active material, the polymer of the present embodiment, a binding resin (binder resin), a conductive auxiliary agent, and the like. The negative electrode may be one in which a layer including a negative electrode material containing a negative electrode active material is formed on a current collector. The negative electrode material may include, in addition to the negative electrode active material, the polymer of the present embodiment, a binding resin (binder resin), a conductive auxiliary agent, and the like.

The electrolyte in the lithium ion battery may be formed from an electrolyte composition. The electrolyte composition may include the polymer of the present embodiment. The electrolyte composition may further include an ion-conductive inorganic solid electrolyte containing lithium ions, an organic solvent, an ionic liquid, and the like. The ion-conductive inorganic solid electrolyte is not particularly limited, and may be an oxide (oxide-based solid electrolyte), a sulfide (sulfide-based solid electrolyte), a hydride (hydride-based solid electrolyte), a halide (halide-based solid electrolyte), or the like. The electrolyte can be formed by applying or laminating the electrolyte composition on the positive electrode or the negative electrode.

The battery may have a separator. The separator may be a porous material, and may be a resin-made porous material. Specific examples include a porous polyolefin membrane, a porous ceramic membrane, and the like.

(Method for producing composition containing organic lithium salt)

As a method for producing the compound (A1), which is an organic lithium salt, the following production method 1 can be mentioned.

Production method 1: A method including a step of reacting a compound (D) represented by the following formula (D) with a compound (E1) represented by the following formula (E1) in the presence of at least two types of lithium salts (also referred to as step 1-2).

[Chem. 39]

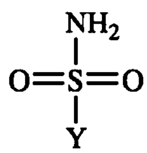

(D)

[Chem. 40]

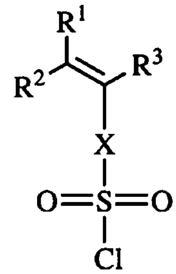

(E1)

Y in formula (D) and X in formula (E1) are selected according to X and Y contained in the desired compound as the compound (A1). When synthesizing the compound (A1'), a compound (E1') represented by the following formula (E1') can be used as the compound (E1).

[Chem. 41]

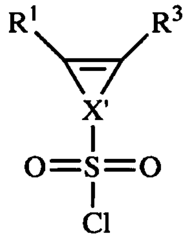

(E1')

The compound (E1') may be a compound (E2) represented by the following formula (E2). Thereby, the compound (A2) can be obtained.

[Chem. 42]

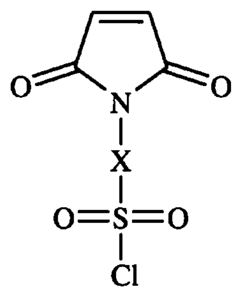

(E2)

The lithium salt is blended into the reaction system as a base. The base may be only a lithium salt. The base does not include a sodium salt such as sodium acetate. Examples of the lithium salt include $Li_2CO_3$, LiOH, and lithium acetate, and $Li_2CO_3$ and LiOH may be used in combination. As a combination of two or more types of lithium salts, a combination of one or more salts of a lithium ion and a strong base and one or more salts of a lithium ion and a weak base can be mentioned. By using two types of lithium salts, side reactions can be suppressed and the desired reaction can be carried out at a sufficient reaction rate.

When two types of lithium salts, a salt of a lithium ion and a strong base and a salt of a lithium ion and a weak base, are used as the lithium salt, the difference in pKa of the conjugate acids of the anions of the two lithium salts is preferably 2 or more, more preferably 3 or more, and even more preferably 5 or more. When three or more types of lithium salts are used, the difference in pKa of the conjugate acids of the anions is checked between each of the lithium salts used, and the minimum value of the differences is preferably 2 or more, more preferably 3 or more, and even more preferably 5 or more.

The reaction temperature in step 1-2 may be, for example, -50°C or higher, may be -30°C or higher, and may be -10°C or higher. The reaction temperature may be 50°C or lower, may be 30°C or lower, and may be 10°C or lower. It may be -50 to 50°C, may be -30 to 30°C, and may be -10 to 10°C. The reaction time may be, for example, 0.1 to 10 hours, may be 0.5 to 8 hours, and may be 1 to 5 hours. Step 1-2 may be performed in a solution. The solvent is not particularly limited, but

examples include an aprotic solvent. Examples of the aprotic solvent include acetonitrile, DMF, DMSO, THF, and the like.

Production method 1 may include a step of synthesizing the compound (E1) before step 1-2 (also referred to as step 1-1). Step 1-1 is not particularly limited, but may be, for example, a step of introducing a - $SO_2Cl$ group into a precursor of the compound (E1). The introduction of the -$SO_2Cl$ group includes, for example, a step of reacting the precursor with chlorosulfonic acid. The reaction temperature in step 1-1 may be, for example, 20 to 80°C, and may be 30 to 60°C. The reaction time may be, for example, 1 to 24 hours, and may be 3 to 18 hours.

As a method for producing the compound (A2), which is an organic lithium salt, the following production method 2 can be mentioned.

Production method 2: A method including a step of reacting a compound (C) represented by the following formula (C) in a solvent in the presence of a lithium-containing base (also referred to as step 2-2). In step 2-2 in production method 2, it is preferable that the counter cation of the base is only a lithium ion. In other words, it is preferable that the base in step 2-2 is only a lithium salt. The base does not include a sodium salt such as sodium acetate.

[Chem. 43]

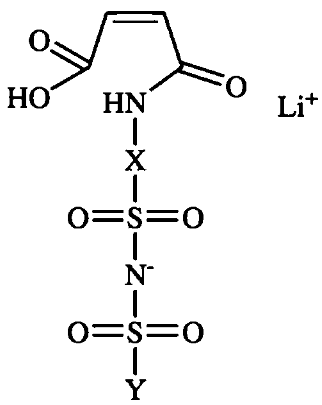

(C)

X and Y in formula (C) can be appropriately selected according to X and Y in the desired compound as the compound (A2).

In production method 2, examples of the lithium-containing base include a lithium salt, and more specifically, lithium carboxylate, lithium acetate, lithium carbonate, and lithium bicarbonate.

The reaction temperature of step 2-2 may be 30 to 100°C, and may be 50°C to 90°C. The reaction time may be 0.1 to 20 hours, may be 0.5 to 10 hours, and may be 1 to 5 hours.

Step 2-2 may be performed in a solution. As the solvent, an aprotic solvent such as 1,4-dioxane may be used.

In production method 2, examples of the solvent include an acid anhydride, for example, acetic anhydride.

A step of synthesizing the compound (C) before step 2-2 (also referred to as step 2-1) may be included.

Patent Literature 1 describes a method for synthesizing the following compound (X). The synthesis method of Patent Literature 1 consists of three steps (see also Comparative Example A1 of the present specification. Note that step 3 in the synthesis method of Patent Literature 1 corresponds to the fourth step in Comparative Example A1 of the present application).

[Chem. 44]

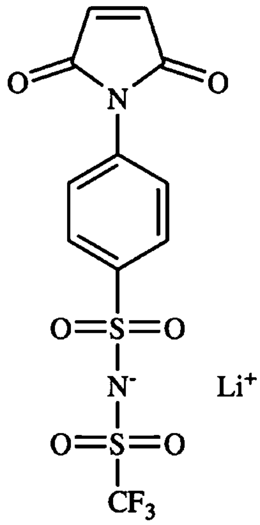

(X)

In step 3 of the synthesis method of Patent Literature 1, the following compound (X1) is reacted in acetic anhydride in the presence of sodium acetate to form a cyclic imide bond to obtain compound (X).

[Chem. 45]

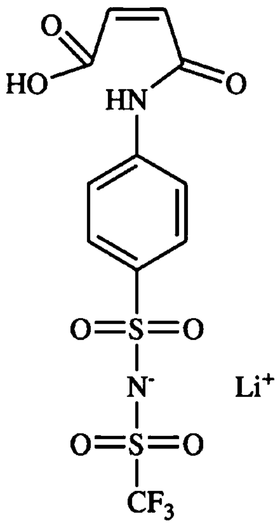

(X1)

Here, as a result of diligent studies by the present inventors, it was found that the product produced by the method of Patent Literature 1 contains a large amount of the sodium salt of compound (X). It is considered that the sodium ions originate from the sodium salt (sodium acetate) in the fourth step. Therefore, when a lithium salt was used instead of the sodium salt, it was found that the synthesis of compound (X) could proceed smoothly and the purity of the lithium salt could be increased (production methods 2 and 3). Furthermore, the method of Patent Literature 1 consists of four steps, and since the product is an ionic compound from the first step, purification, handling, and the like are not easy. Therefore, in view of shortening the synthesis route and introducing an ionic functional group, production method 1 was achieved. As described above, according to the method of the present disclosure, a high-purity lithium salt (that is, the composition of the present embodiment) can be obtained. Furthermore, if an alkali metal other than lithium is introduced once in the process of synthesizing a lithium salt, it is necessary to perform cation exchange using a dialysis membrane or the like in order to increase the lithium concentration in the lithium salt (or a composition containing the desired lithium salt and a salt substituted with another alkali metal). However, performing cation exchange is not preferable because it increases the number of steps, making the process more complicated, and increases the production cost of the lithium salt. In addition, since cation exchange is generally performed in an aqueous solution, when producing a compound having a functional group that is easily hydrolyzed, such as the maleimide group exemplified this time, the target compound decomposes, so it is very difficult to perform cation exchange treatment in the first place and subsequently increase the lithium ion concentration.

Although several embodiments have been described above, the present disclosure is not limited in any way to the above embodiments. Furthermore, the descriptions of the embodiments described above can be applied to each other.

## Examples

(Example A1)

The above compound (X) (monomer of Example A1) and a copolymer were synthesized by the following method.

<First step: Synthesis of intermediate 1>

Under a nitrogen atmosphere, trifluoromethanesulfonamide (52.5 mmol, 7.83 g, manufactured by Tokyo Chemical Industry Co., Ltd.) was dissolved in dehydrated acetonitrile (150 mL, manufactured by Kanto Chemical Co., Inc.). To this solution, lithium hydroxide (105 mmol, 2.51 g, manufactured by Tokyo Chemical Industry Co., Ltd.) and 4-acetamidobenzenesulfonyl chloride (50 mmol, 11.68 g, manufactured by Tokyo Chemical Industry Co., Ltd.) were added in this order and heated under reflux for 5 hours. After cooling to room temperature, an excess amount of acetonitrile (700 mL) was added to precipitate a solid, which was separated by filtration. Then, the filtrate was dried under reduced pressure, and the obtained solid was washed with dichloromethane (manufactured by Kanto Chemical Co., Inc.) to obtain intermediate 1. The yield was 97.1%.

- Structural formula of intermediate 1:

[Chem. 46]

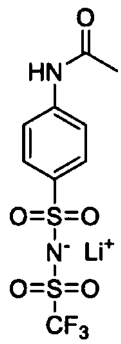

<Second step: Synthesis of intermediate 2>

Under a nitrogen atmosphere, 5% hydrochloric acid (22.5 mL) was added to intermediate 1 (15 mmol, 5.28 g), and the mixture was stirred at 90°C for 2 hours. After cooling to room temperature, an aqueous lithium hydroxide solution was added until the pH became 7 or higher while checking with pH test paper or the like, and then a solid was obtained by drying under reduced pressure. The obtained solid was extracted with an acetonitrile solution and dried under reduced pressure to obtain intermediate 2. The yield was 92.6% based on the raw material of the intermediate 1.

- Structural formula of intermediate 2:

[Chem. 47]

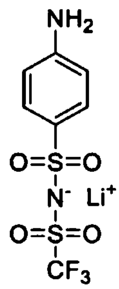

<Third step: Synthesis of intermediate 3>

Under a nitrogen atmosphere, maleic anhydride (13.3 mmol, 1.30 g, manufactured by Tokyo Chemical Industry Co., Ltd.) was dissolved in dehydrated 1,4-dioxane (manufactured by Kanto Chemical Co., Inc.). To this solution, a solution of intermediate 2 (13.2 mmol, 4.09 g) in dehydrated tetrahydrofuran (26.4 mL, manufactured by Kanto Chemical Co., Inc.), prepared under a nitrogen atmosphere, was added dropwise in its entirety, and the mixture was stirred at room temperature for 12 hours. After the reaction, the mixture was dried under vacuum at 60°C for 4 hours to obtain a solid containing intermediate 3.

- Structural formula of intermediate 3:

[Chem. 48]

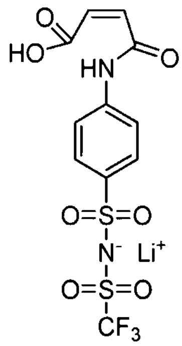

<Fourth step: Synthesis of compound (X)>

Under a nitrogen atmosphere, a solid containing intermediate 3 (14.0 mmol, 5.70 g) and an aqueous lithium acetate solution (14.0 mmol, 0.92 g) were added to acetic anhydride (12.3 mL, manufactured by Tokyo Chemical Industry Co., Ltd.), and the mixture was stirred at 70°C for 3 hours. The entire reaction solution was added dropwise to an excess amount of diethyl ether (manufactured by Kanto Chemical Co., Inc.) at 0°C, and the precipitate was collected by filtration. Under an inert atmosphere, the precipitate was extracted with dehydrated acetonitrile (manufactured by Kanto Chemical Co., Inc.) and dried under reduced pressure to obtain the monomer of Example A1. The overall yield through all steps was 72.8%.

(Synthesis of copolymer)

Monomer of Example A1: 0.558 g, styrene: 0.149 g, and azobisisobutyronitrile: 11.7 mg were dissolved in 6.7 mL of dehydrated acetonitrile, and tetralin was added as an internal standard substance. The reaction was carried out at 60°C for 24 hours under a nitrogen atmosphere while confirming the monomer consumption rate. The polymerization solution was dialyzed in acetonitrile and dried under vacuum at 120°C to obtain 0.640 g (yield 87%) of a copolymer (copolymer of Example A1). The monomer incorporation ratio was monomer of Example A1:styrene = 52:48. The monomer incorporation ratio was calculated from $^1$H-NMR of the copolymer.

The obtained copolymer had a number average molecular weight Mn = $9.4\times10^4$, a weight average molecular weight Mw = $4.2\times10^5$, and a molecular weight distribution Mw/Mn = 4.45. The number average molecular weight and weight average molecular weight were measured by gel permeation chromatography.

<Measurement of lithium ion transference number>

An electrolyte composition was produced by mixing 100 parts by mass of the above copolymer, 50 parts by mass of poly(vinylidene fluoride-co-hexafluoropropylene) (PVDF-HFP), and 300 parts by mass of an organic solvent (ethylene carbonate:propylene carbonate = 1:1 (volume ratio)) (Kishida Chemical Co., Ltd.).

The lithium ion transference number of the above electrolyte composition was measured by the following method. Three samples for measurement were prepared, and the lithium ion transference number was measured for each. The results are shown in Table 1.

Lithium ion transference number:

An evaluation cell of a CR2032 coin-type lithium battery was assembled in a glove box under a dry argon atmosphere. Specifically, a test laminate was produced by laminating each layer in the following order in the evaluation cell. (Lithium/electrolyte composition/lithium)

The method for measuring the lithium ion transference number is the one introduced in Polymer, 28, 2324 (1987). That is, at room temperature (25°C), 10 mV was applied to the test laminate, the initial current value ($I_0$) and the steady current value ($I_{ss}$) were measured, and further, the interface resistance measurement value $R_0$ before voltage application and the interface resistance measurement value $R_{SS}$ after voltage application were determined by the complex impedance method. Then, the obtained values were introduced into the following formula to determine the lithium ion transference number ($t_{Li+}$). In the formula, V is the applied voltage.

$$t_{Li+} = I_{ss}(V - I_0R_0) / I_0(V - I_{SS}R_{SS})$$

Fig. 1 shows the measurement results of the lithium ion transference number for one sample of the electrolyte composition containing the copolymer of Example A1.

(Comparative Example A1)

The monomer of Comparative Example A1 was obtained by performing the reaction and production in the same manner as in Example A1, except that an aqueous sodium acetate solution (13.3 mmol, 1.09 g, manufactured by Tokyo Chemical Industry Co., Ltd.) was used instead of the aqueous lithium acetate solution in the fourth step of Example A1. The synthesis method for the monomer of Comparative Example 1A corresponds to the synthesis method of Patent Literature 1. In the same manner as in Example A1, the copolymer of Comparative Example A1 was produced using the monomer of Comparative Example A1, and the lithium ion transference number was measured.

The products obtained in Example A1 and Comparative Example A1 were each analyzed by an ICP (inductively coupled plasma) atomic emission spectrometry apparatus. From the measurement results, the respective amounts of substance of sodium and lithium were calculated with half the amount of substance of sulfur contained in the product as 1 (that is, the molar ratio of lithium and sodium to the sulfonylimide group was calculated). The same analysis was also performed on intermediate 2 and the copolymers of Example A1 and Comparative Example A1 to calculate the respective contents of sodium and lithium. The results are shown in Table 1.

[Table 1]

| IPC analysis | | Comparative Example A1 (Synthesis method of Patent Document 1) | | | Example Al | | |
|---|---|---|---|---|---|---|---|
| | | Intermediate 1 | Monomer | Copolymer | Intermediate 1 | Monomer | Copolymer |
| Molar ratio | Lithium content | 1.3 | 0.1 | <0.1 | 1.3 | 1.3 | 1.2 |
| | Sodium content | 0.2 | 1.3 | 1.2 | 0.2 | 0.4 | 0.3 |
| Lithium ion transference number | | - | - | 0.56 to 0.74 | - | - | 0.88 to 0.91 |

Regarding the synthesis method of intermediate 1, the method of Example A1 and the method of Comparative Example A1 are common, so the same result was obtained. In the method of Example A1, the purity of the lithium salt was maintained even in the monomer of Example A1, which is the final product. In addition, the copolymer obtained using this monomer also maintained a sufficient lithium content. On the other hand, in Comparative Example A1, where sodium acetate was used in the fourth step, most of the monomer was converted to the sodium salt.

Furthermore, in the electrolyte composition using the copolymer produced using the monomer of Example A1, the lithium ion transference number was high, and the numerical values were stable over three measurements. On the other hand, in the electrolyte composition using the copolymer produced using the monomer of Comparative Example A1, the lithium ion transference number was low compared to that of Example A1, and the measured values were also not stable. Fig. 2 shows the measurement results of the lithium ion transference number for one sample of the electrolyte composition containing the copolymer of Comparative Example A1.

(Example B1)

The monomer of Example B1 was produced by the following method.

<Step 1: Synthesis of intermediate B1>

At 0°C, chlorosulfonic acid (69 mL, 1.04 mol) was added to N-phenylmaleimide (30 g, 0.17 mol), and the mixture was stirred at 45 to 50°C for 1 hour. The resulting product was cooled to room temperature and poured onto ice. After stirring for a while, the precipitated crystals were collected by filtration, and the obtained crystals were purified by silica gel column chromatography (hexane/ethyl acetate = 2/1 to 1/1 (volume ratio)), resulting in 34 g (yield 73%) of intermediate B1 as a pale yellow solid.

- Structural formula of intermediate B1:

[Chem. 49]

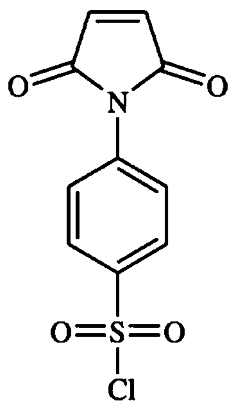

Under a nitrogen atmosphere, trifluoromethanesulfonamide (manufactured by Tokyo Chemical Industry Co., Ltd.) (4.22 g, 28.3 mmol) was dissolved in dehydrated acetonitrile (160 mL, manufactured by Kanto Chemical Co., Inc.). To this solution, 1.0 equivalent of lithium carbonate, 1.2 equivalents of lithium hydroxide (manufactured by Tokyo Chemical Industry Co., Ltd.), and intermediate B1 were added in this order with respect to trifluoromethanesulfonamide, and the reaction was carried out at 0°C for 4.5 hours. After returning to room temperature, the mixture was filtered, and the solvent was distilled off from the filtrate under reduced pressure. The residue was subjected to decantation with diethyl ether and dried under reduced pressure, resulting in 10.5 g of the target compound as a pale brown solid. The product was analyzed by $^1$H-NMR. The results are shown in Table 2.

(Examples B2 to B4, Comparative Examples B1 to B4)

Synthesis was performed in the same manner as in Example B1 to obtain a product, except that the amount of lithium salt used and the reaction conditions (reaction temperature and reaction time) were changed as shown in Table 2. The product was analyzed by $^1$H-NMR. The results are shown in Table 2.

[Table 2]

| Example No. | Lithium salt (amount used) | Reaction conditions | NMR analysis results |
|---|---|---|---|
| Comparative Example B1 | $Li_2CO_3$ (2.0 eq.) | Reflux, 5 hours | No reaction. |
| Comparative Example B2 | LiOH (2.0 eq.) | Reflux, 4 hours | Complex mixture |
| Comparative Example B3 | LiOH (2.0 eq.) | 0°C, 3 hours | Compound (X) and side products |
| Comparative Example B4 | LiOH (2.0 eq.) | -20°C, 3 hours | Compound (X) and side products |
| Example B1 | $Li_2CO_3$ (1.2 eq.)<br>LiOH (1.0 eq.) | 0°C, 3 hours | Compound (X) and approx. 25 mol% of unreacted material and side products |
| Example B2 | $Li_2CO_3$ (1.0 eq.)<br>LiOH (1.5 eq.) | 0°C, 3 hours | Compound (X) and approx. 25 mol% of unreacted material and side products |
| Example B3 | $Li_2CO_3$ (1.0 eq.)<br>LiOH (1.8 eq.) | 0°C, 3 hours | Compound (X) and approx. 10 mol% of unreacted material and side products |
| Example B4 | $Li_2CO_3$ (1.0 eq.)<br>LiOH (2.0 eq.) | 0°C, 3 hours | Compound (X) only |

In Comparative Example B1, where only lithium carbonate was used as the lithium salt, the reaction did not proceed, and the target product (compound (X)) could not be confirmed by $^1H$-NMR.

In Comparative Example 2B, where only lithium hydroxide was used as the lithium salt and the reaction was carried out while refluxing, the reaction proceeded, but a complicated mixture containing many side reaction products was obtained. Many peaks were observed in the $^1H$-NMR spectrum, and identification of the side products was difficult, and since many peaks were overlapping, the formation of compound (X) could not be confirmed.

In Comparative Example B3 and Comparative Example B4, where the reaction temperature was lower than in Comparative Example 2B, the formation of compound (X) could be confirmed, but they contained such a large amount of by-products that its content could not be specified.

On the other hand, in Examples B1 to B3, the reaction proceeded smoothly, and contained about 10 to 25 mol% of unreacted material and side reaction products (one in which the maleimide group of intermediate B1 was ring-opened) with respect to 100 mol% of compound (X). In Example B4, unreacted material and side reaction products were not observed, and only compound (X) was obtained. Although the detailed reaction mechanism has not been elucidated, it is presumed that a complex salt is formed by using a strongly basic salt and a weakly basic salt in combination. Furthermore, from Examples B2 to B4, it is presumed that by changing the amount of LiOH, which is a strong base, the basicity of the complex salt changed, and the basicity became optimal for the reaction under the conditions of Example B4.

## Claims

1. A method for producing a compound (A1) represented by the following formula (A1), the method comprising: a step of reacting a compound (D) represented by the following formula (D) with a compound (E1) represented by the following formula (E1) in the presence of at least two types of lithium salts.

[Chem. 1]

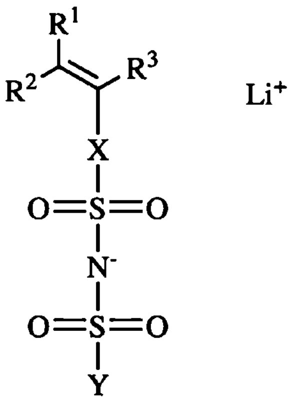

(A1)

(In formula (A1), $R^1$ is a hydrogen atom or a monovalent substituent, $R^3$ is a hydrogen atom or a monovalent substituent, Y is a halogen atom or a monovalent organic group having 1 to 20 carbon atoms, and X and $R^2$ satisfy the following (1) or (2):

(1) X is a divalent organic group having 1 to 20 carbon atoms, and $R^2$ is a monovalent organic group.
(2) $R^2$ and X are taken together to form a trivalent group.)

[Chem. 2]

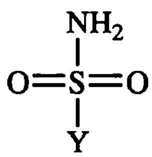

(D)

[Chem. 3]

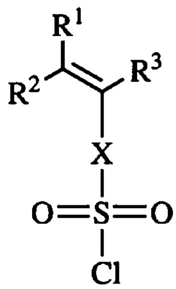

(E1)

2. The method for producing according to claim 1, wherein the compound (A1) comprises a compound (A2) represented by the following formula (A2).

[Chem. 4]

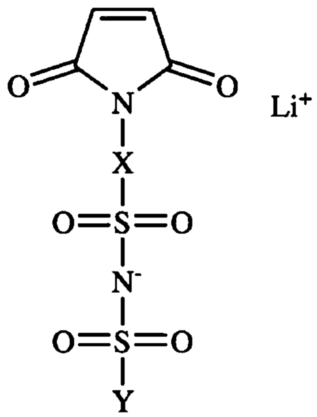

(A2)

3. A method for producing a compound (A2) represented by the following formula (A2), the method comprising: a step of reacting a compound (C) represented by the following formula (C) in a solvent in the presence of a lithium-containing base.

[Chem. 5]

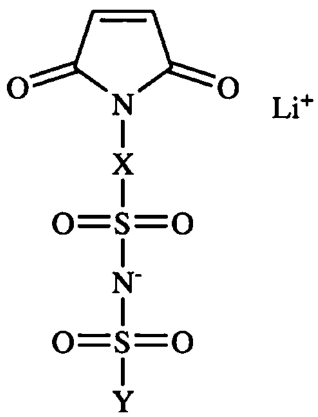

(A2)

(In formula (A2), X is a divalent organic group having 1 to 20 carbon atoms, and Y is a halogen atom or a monovalent organic group having 1 to 20 carbon atoms.)

[Chem. 6]

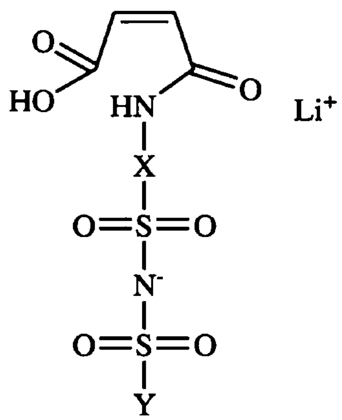

(C)

4. The method for producing according to claim 3, wherein the lithium-containing base comprises lithium acetate.

5. The method for producing according to claim 3 or 4, wherein the solvent comprises acetic anhydride.

6. A composition comprising a compound (A) represented by the following formula (A), wherein a proportion of the compound (A) is 50 mol% or more with respect to a total amount of substance of the compound (A) and a compound (B) represented by the following formula (B) in the composition.

[Chem. 7]

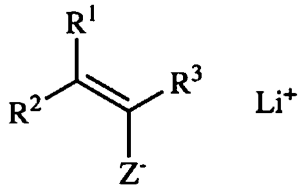

(A)

(In formula (A), $R^1$ is a hydrogen atom or a monovalent substituent, $R^3$ is a hydrogen atom or a monovalent substituent, and $R^2$ and $Z^-$ satisfy the following (3) or (4):

(3) $R^2$ is a hydrogen atom or a monovalent substituent, and $Z^-$ is a monovalent group having a monovalent anionic functional group.
(4) $R^2$ and $Z^-$ are taken together to form a divalent group having a monovalent anionic functional group.)

[Chem. 8]

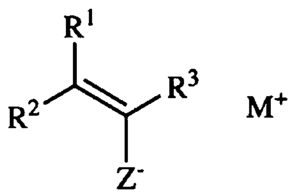

(B)

(In formula (B), $R^1$ to $R^3$ and $Z^-$ are the same as $R^1$ to $R^3$ and $Z^-$ in formula (A), respectively, and $M^+$ is a monovalent cation other than $H^+$ and $Li^+$.)

7. The composition according to claim 6, wherein the compound (A) comprises a compound (A1) represented by the following formula (A1), and the compound (B) comprises a compound (B1) represented by the following formula (B1).

[Chem. 9]

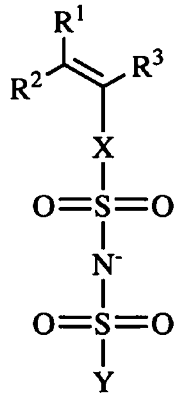

Li⁺ (A1)

(In formula (A1), Y is a halogen atom or a monovalent organic group having 1 to 20 carbon atoms, and X and $R^2$ satisfy the following (1) or (2):

(1) X is a divalent organic group having 1 to 20 carbon atoms, and $R^2$ is a monovalent organic group.
(2) $R^2$ and X are taken together to form a trivalent group.)

[Chem. 10]

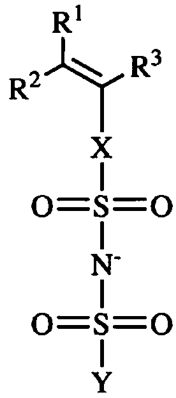

$M^+$ (B1)

(In formula (B1), X and Y are the same as X and Y in formula (A1), respectively.)

**8.** The composition according to claim 6, wherein the compound (A) comprises a compound (A2) represented by the following formula (A2), and the compound (B) comprises a compound (B2) represented by the following formula (B2).

[Chem. 11]

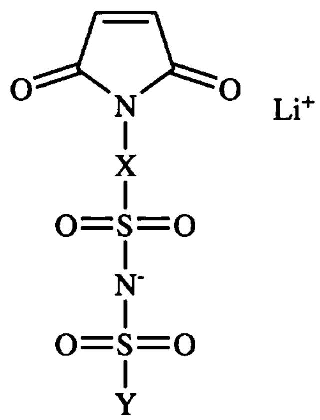

(A2)

(In formula (A2), X is a divalent organic group having 1 to 20 carbon atoms, and Y is a halogen atom or a monovalent organic group having 1 to 20 carbon atoms.)

[Chem. 12]

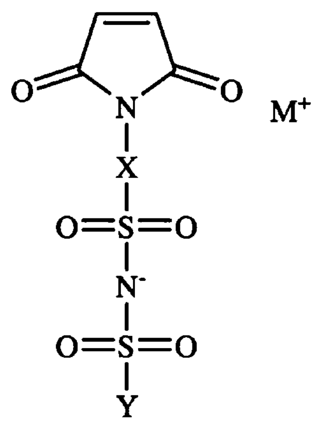

(B2)

(In formula (B2), X and Y are the same as X and Y in formula (A2), respectively.)

**9.** The composition according to any one of claims 6 to 8, which is a monomer composition for forming a polymer for an electrolyte of a lithium ion battery.

**10.** A polymer comprising a structural unit (PA) represented by the following formula (PA), wherein a proportion of the structural unit (PA) is 50 mol% or more with respect to a total amount of substance of the structural unit (PA) and a structural unit (PB) represented by the following formula (PB) in the polymer.

[Chem. 13]

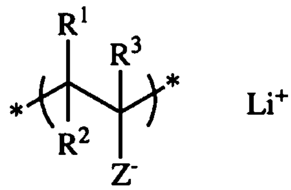

(PA)

(In formula (PA), $R^1$ is a hydrogen atom or a monovalent substituent, $R^3$ is a hydrogen atom or a monovalent substituent, and $R^2$ and $Z^-$ satisfy the following (3) or (4):

(3) $R^2$ is a hydrogen atom or a monovalent substituent, and $Z^-$ is a monovalent group having a monovalent anionic functional group.
(4) $R^2$ and $Z^-$ are taken together to form a divalent group having a monovalent anionic functional group.)

[Chem. 14]

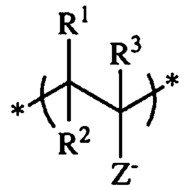

$M^+$ (PB)

(In formula (PB), $R^1$ to $R^3$ and $Z^-$ are the same as $R^1$ to $R^3$ and $Z^-$ in formula (PA), respectively, and $M^+$ is a monovalent cation other than $H^+$ and $Li^+$.)

**11.** The polymer according to claim 10, wherein the structural unit (PA) comprises a structural unit (PA1) represented by the following formula (PA1), and the structural unit (PB) comprises a structural unit (PB1) represented by the following formula (PB1).

[Chem. 15]

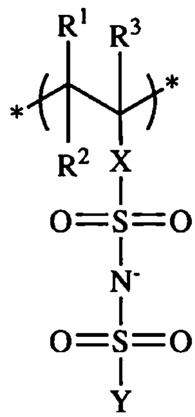

$Li^+$ (PA1)

(In formula (PA1), Y is a halogen atom or a monovalent organic group having 1 to 20 carbon atoms, and X and $R^2$ satisfy the following (1) or (2):

(1) X is a divalent organic group having 1 to 20 carbon atoms, and $R^2$ is a monovalent organic group.
(2) $R^2$ and X are taken together to form a trivalent group.)

[Chem. 16]

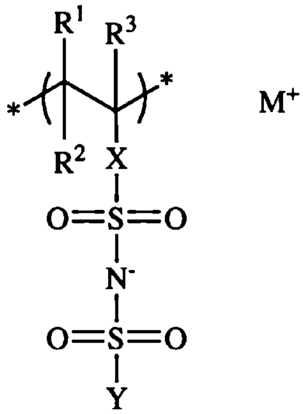

(PB1)

(In formula (PB1), X and Y are the same as X and Y in formula (PA1), respectively.)

**12.** The polymer according to claim 10, wherein the structural unit (PA) comprises a structural unit (PA2) represented by the following formula (PA2), and the structural unit (PB) comprises a structural unit (PB2) represented by the following formula (PB2).

[Chem. 17]

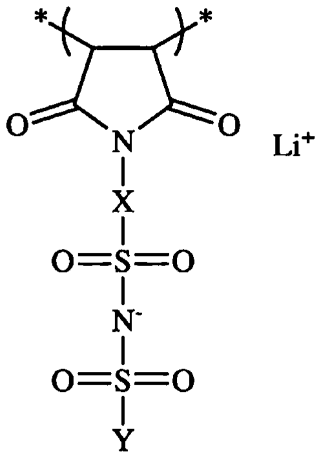

(PA2)

(In formula (PA2), X is a divalent organic group having 1 to 20 carbon atoms, and Y is a halogen atom or a monovalent organic group having 1 to 20 carbon atoms.)

[Chem. 18]

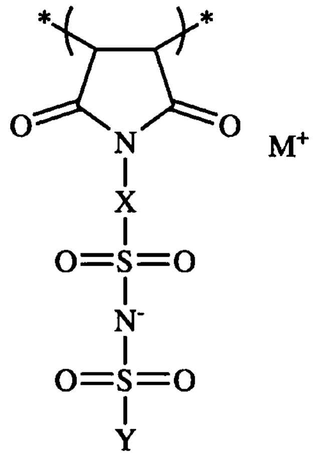

(PB2)

(In formula (PB2), X and Y are the same as X and Y in formula (PA2), respectively.)

**13.** An electrolyte composition comprising the polymer according to any one of claims 10 to 12.

**14.** A battery comprising the electrolyte composition according to claim 13.

*Fig.1*

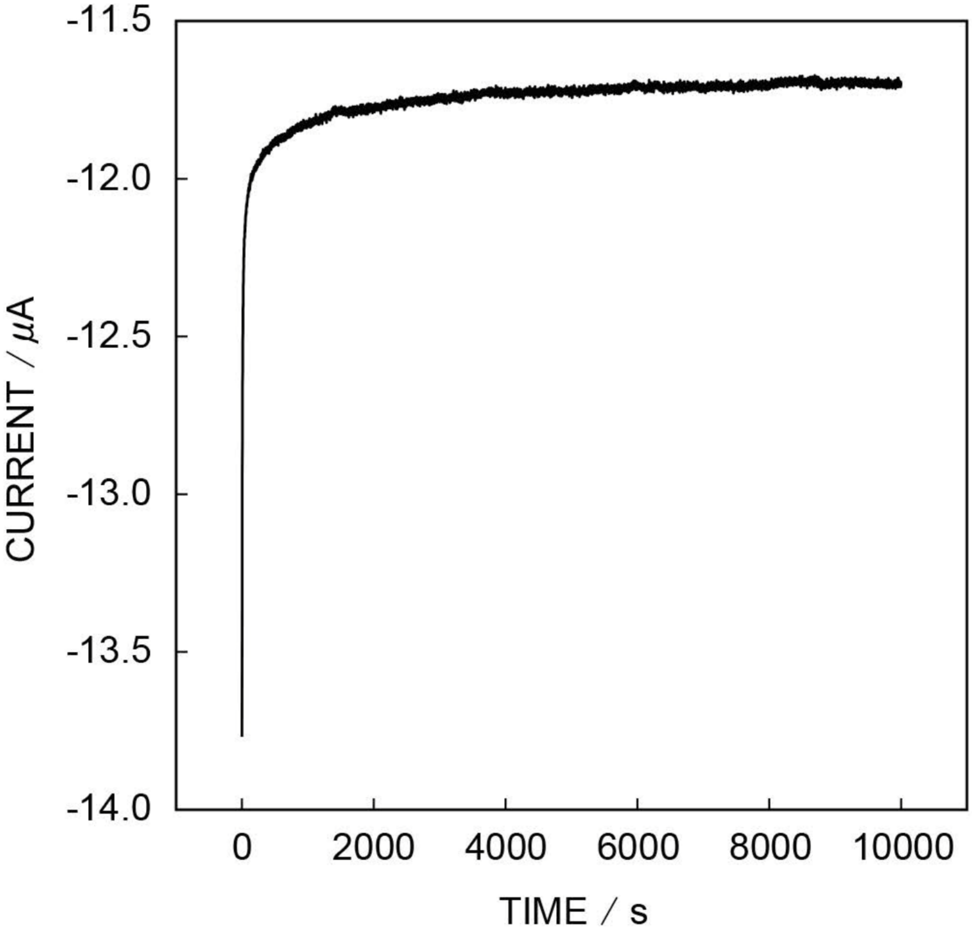
-11.5
-12.0
-12.5
-13.0
-13.5
-14.0
CURRENT / μA
0
2000
4000
6000
8000
10000
TIME / s

# Fig.2

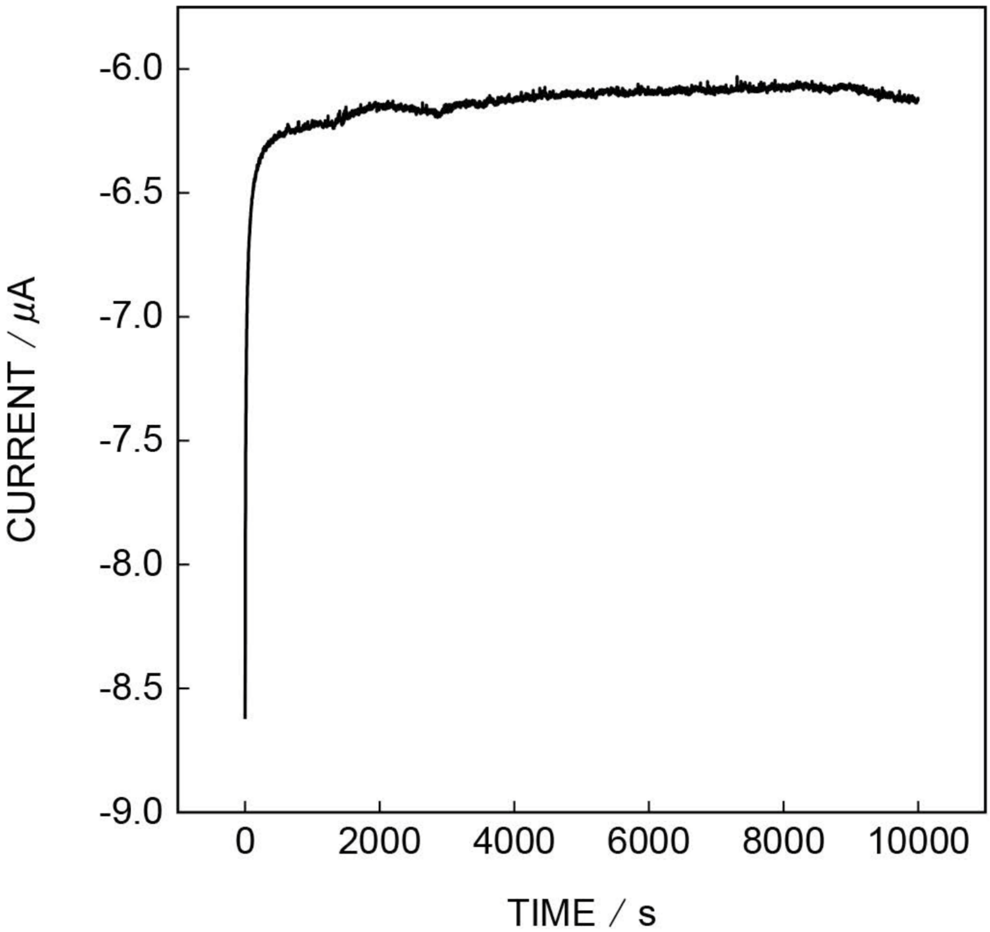
-6.0
-6.5
-7.0
-7.5
-8.0
-8.5
-9.0
CURRENT / μA
0
2000
4000
6000
8000
10000
TIME / s

## INTERNATIONAL SEARCH REPORT

International application No. **PCT/JP2024/016899**

### A. CLASSIFICATION OF SUBJECT MATTER

***C08F 22/40***(2006.01)i; ***C07D 207/452***(2006.01)i; ***H01M 10/052***(2010.01)i; ***H01M 10/0565***(2010.01)i
FI: C08F22/40; C07D207/452; H01M10/052; H01M10/0565

According to International Patent Classification (IPC) or to both national classification and IPC

### B. FIELDS SEARCHED

Minimum documentation searched (classification system followed by classification symbols)

C08F22/40; C07D207/452; H01M10/052; H01M10/0565

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| X<br>A | WO 2015/029248 A1 (HITACHI, LTD.) 05 March 2015 (2015-03-05)<br>claims, paragraphs [0039]-[0062], examples | 6-7, 9-11<br>1-5, 8, 12-14 |
| A | JP 2021-088695 A (BELENOS CLEAN POWER HOLDING AG) 10 June 2021 (2021-06-10)<br>entire text | 1-14 |
| A | JP 2014-529863 A (UNIVERSITE D'AIX-MARSEILLE) 13 November 2014 (2014-11-13)<br>entire text | 1-14 |
| P, A | JP 2023-077153 A (SUMITOMO CHEMICAL COMPANY, LIMITED) 05 June 2023 (2023-06-05)<br>entire text | 1-14 |

☐ Further documents are listed in the continuation of Box C.   ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **11 July 2024** | **30 July 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**
**Information on patent family members**

International application No.
**PCT/JP2024/016899**

| Patent document cited in search report | Publication date (day/month/year) | Patent family member(s) | Publication date (day/month/year) |
|---|---|---|---|
| WO 2015/029248 A1 | 05 March 2015 | (Family: none) | |
| JP 2021-088695 A | 10 June 2021 | US 2021/0163649 A1<br>entire text<br>EP 3832757 A1<br>CN 112898457 A<br>KR 10-2021-0070189 A<br>TW 202126702 A<br>KR 10-2022-0129515 A | |
| JP 2014-529863 A | 13 November 2014 | US 2014/0272600 A1<br>entire text<br>WO 2013/034848 A1<br>EP 2753656 A1<br>FR 2979630 A1<br>CA 2846267 A<br>CN 103874724 A<br>KR 10-2014-0061501 A<br>ES 2560406 T | |
| JP 2023-077153 A | 05 June 2023 | (Family: none) | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2018176134 A